(19) **Europäisches Patentamt**

**European Patent Office**

**Office européen des brevets**

(11) **EP 1 061 932 B1**

(12) **EUROPEAN PATENT SPECIFICATION**

(45) Date of publication and mention
of the grant of the patent:
**25.01.2006 Bulletin 2006/04**

(21) Application number: **98953095.1**

(22) Date of filing: **19.11.1998**

(51) Int Cl.:
*A61K 35/32* (2006.01)     *C07C 69/587* (2006.01)
*C07C 67/00* (2006.01)

(86) International application number:
**PCT/KR1998/000368**

(87) International publication number:
**WO 1999/026640 (03.06.1999 Gazette 1999/22)**

(54) **PHARMACEUTICAL COMPOSITION CONTAINING EXTRACTS OF CERVUS NIPPON ANTLERS HAVING GROWTH-STIMULATING ACTIVITIES OF HEMATOPOIETIC STEM CELLS AND MEGAKARYOCYTES**

PHARMAZEUTISCHE ZUSAMMENSETZUNG ENTHALTEND GEWEIHEXTRAKTE VON CERVUS NIPPON MIT WACHSTUMSSTIMULIERENDER AKTIVITÄT AUF HEMATOPOIETISCHE STAMMZELLEN UND MEGAKARYOZYTEN

COMPOSITION PHARMACEUTIQUE CONTENANT DES EXTRAITS DE BOIS DE CERVUS NIPPON POSSEDANT UNE ACTIVITE DE STIMULATION DE LA CROISSANCE DES CELLULES SOUCHES HEMATOPOIETIQUES ET DES MEGACARYOCYTES

(84) Designated Contracting States:
**CH DE ES FI FR GB IT LI SE**

(30) Priority: **20.11.1997 KR 9761346**
**17.01.1998 KR 9802073**
**30.07.1998 KR 9830819**

(43) Date of publication of application:
**27.12.2000 Bulletin 2000/52**

(73) Proprietors:
• **Jhon, Gil Ja**
**Seoul (KR)**
• **Han, So Yeop**
**Seoul (KR)**
• **Kim, Sang Hee**
**Seoul (KR)**

(72) Inventors:
• **Jhon, Gil Ja**
**Daehyun-dong, Seodaemoon-ku, Seoul (KR)**
• **Yang, Hyun Ok**
**388-1,Poongnap-dong,Songpa-ku,Seoul (KR)**
• **Kim, Yang Mi**
**Mojin-dong, Kwangjin-ku, Seoul (KR)**
• **Kim, Sang Hee**
**Songpa-ku, Seoul (KR)**
• **Shu, Gi Young**
**11-1, Daehyun-dong, Seodaemoon-ku, Seoul (KR)**

• **Han, So Yeop**
**11-1, Daehyun-dong, Seodaemoon-ku, Seoul (KR)**

(74) Representative: **Dost, Wolfgang et al**
**Patent- und Rechtsanwälte**
**Bardehle . Pagenberg . Dost .**
**Altenburg . Geissler**
**Galileiplatz 1**
**81679 München (DE)**

(56) References cited:
• **DATABASE WPI ON EPOQUE, Week 9116, LONDON: DERWENT PUBLICATIONS LTD., AN 91-112630; & JP 3052818 A (MORISHITA JINTAN KK) 07 March 1991.**
• **DATABASE WPI ON EPOQUE, Week 9748, LONDON: DERWENT PUBLICATIONS LTD., AN 97-521873; & JP 9249576 A (TANAKA) 22 September 1997.**
• **DATABASE WPI ON EPOQUE, Week 9729, LONDON: DERWENT PUBLICATIONS LTD., AN 97-311393; & CN 1104897 A (XU S) 12 July 1995.**
• **CHEMICAL ABSTRACTS, Vol. 120, No. 11, 14 March 1994, (Columbus, Ohio, USA), page 869, Abstract No. 132660w, SOFTLY et al., "Composition of Representative SALATRIM Fat Preparations"; & J. AGRIC. FOOD CHEM., 1994, 42(2), 461-467 (Eng).**

- CHEMICAL ABSTRACTS, Vol. 110, No. 23, 05 June 1989, (Columbus, Ohio, USA), page 394, Abstract No. 218804b, NISHIDA Y., "Hair Growth Stimulating Tonics Containing Fatty Acid Glyceride Acetates"; & JP 1009912 A (13 January 1989).

- CHEMICAL ABSTRACTS, Vol. 99, No. 21, 21 November 1983, (Columbus, Ohio, USA), page 324, Abstract No. 172219p, TAKAHASHI et al., "Chromatographic Rules in Elution of Individual Molecular Species of Triglyceride"; & NIPPON SUISAN GAKKAISHI, 1983, 49(8), 1301 (Eng).

**EP 1 061 932 B1**

**Description**

**TECHNICAL FIELD**

The present invention relates to a pharmaceutical composition containing extracts of *Cervus nippon* antlers having growth-stimulating activities of hematopoietic stem cells and megakaryocytes. More specifically, the present invention relates to a pharmaceutical composition having growth-stimulating activities of hematopoietic stem cells and megakaryocytes, containing as an active component chloroform extracts of *Cervus nippon* antler (hereinafter referred to as "extract CN-C") which are obtained by extracting *Cervus nippon* antler with hexane and further extracting the residue of the hexane extract with chloroform, and if necessary, fractionating and purifying the chloroform extract of *Cervus nippon* antler by silica gel column chromatography and high performance thin layer chromatography (HPTLC).

**BACKGROUND ART**

In traditional oriental medicine in Korea, deer antler together with ginseng have been widely used for their various acclaimed medicinal effects. The deer species from which the antlers are harvested belong to *Cornu cervi parvum* and include *Cervus nippon* Temminick var manturicus Swinhoe and *Cervus elephus* L. The antlers are harvested before they are cornified and dried, and uncornified testa shells are boiled in water along with other herbal medicines for use.

*Cervus nippon* antler has been acclaimed to have numerous medicinal effects, e.g., a tonic, growth- and development-promoting effects, promoting hematopoietic function, treating nervous breakdown, beneficial to cardiac insufficiency, generally improving the function of the five viscera and six entrails (Dong-euibogam, a classical medical literature in Korea). Other literatures in traditional medicine, concerning the effects of deer antlers, also report a tonic, nourishing effect, a powerful general systemic effects including improving cardiac function, a fatigue recovery effects, enhancing systemic resistance to various diseases.

Numerous previous investigation were performed to identify the active component(s) of the "mysterious" broad beneficial medicinal effects of deer antlers. Many substances were reportedly identified *Cervus nippon* antler and were claimed to be the active ingredient at least in part. These are : certain free amino acids, trace metallic elements, hexose, pentose, hexosamine, uronic acid, sialic acid, acid mucopolysacharides such as hyaluronic acid, chondroitin A, various fatty acids, and prostaglandins. Further, it has been reported that glycolipid, phospholipid, cholesterol hypoxanthine, cholest-5-ene-3β,7α-diol, cholesterol ester, polyamine were isolated from *Cervus nippon* antler. Others reported the presence of estrone and estradiol receptor. These reports, however, did not specifically demonstrate biological effects of certain chemical components of *Cervus nippon* antlers. None of these reports have also chemically identified and synthesized them *in vitro* for potential pharmaceutical production.

Chemotherapy and radiotherapy of malignant neoplastic diseases result not only in damages to cancer cells, which are desired result, but also normal cells which are to be preserved and protected. Especially hematopoietic cells, which provide blood cells as well as the first line of defense against infections, are very sensitive to those treatments.

Therefore, myelosuppression and immunosuppression from vigorous anti-cancer chemotherapy, rather than underlying malignant disease, may lead to death of patients, and these toxic effects have been limiting factors in more aggressive anti-cancer treatment. There has been a strong need for pharmaceutical agents which can be used for protection and/or restoration of hematopoietic and immune systems during the cytotoxic anti-cancer chemotherapy and radiotherapy.

Any drugs which stimulate growth and differentiation of hematopoietic stem cells would be used for such purpose. Such drugs may be used in other disease conditions where hematopoietic stem cells are abnormally suppressed or nonfunctional, as in aplastic anemia, cyclic neutropenia, congenital immune deficiency syndrome, anemia from chronic renal failure, infections due to myelosuppression and decreased immunity in elderly population.

There have been a significant development in the understanding of myelopoiesis and various factors affecting several steps in hematopoiesis. Examples of the growth factors which are already developed and commercialized include granulocyrte macrophage colony stimulating factor(GM-CSF), granulocyrte colony stimulating factor(G-CSF) and erythropoietin(EPO). All of these are, however, a kind of cytokines, effective only in injectable form, they have some undesirable side reactions, their effect is very short lived, and still very expensive. There is still a strong need for a less expensive drug, and preferably effective with oral administration.

The present inventors have experienced several interesting clinical cases of unexplained rapid restoration of suppressed bone marrow after cancer chemotherapy. On close questioning it was suggested that the unexplained preservation of bone marrow or rapid restoration after suppression might have been related to the fact that the patients had taken deer antler extracts, for in Korea it is not unusual that many patients combine traditional oriented medicines for the treatments given by physicians.

3

## DISCLOSURE OF INVENTION

The present inventors therefore investigated the possibility of certain active ingredients being present in *Cervus nippon* antlers and looked for bone marrow stem cell stimulating effects of those antler extracts. As a result, we have identified that chloroform extracts of the *Cervus nippon* antler (hereinafter designated as "Extract CN-C") indeed has growth-stimulating effects on hematopoietic stem cells and magakaryocytes.

The present inventors recognized that these purified active compounds from the extract CN-C or their derivatives have the biological effects of stimulating hematopoietic stem cells, megakaryocytes, monocyte, and lymphocytes and that these compounds are effective not only after parenteral administration, but uniquely effective on oral administration.

It is an object of the present invention to provide a pharmaceutical composition having growth-stimulating activities of hematopoietic stem cells and megakaryocytes comprising extracts of *Cervus nippon* antlers as an active component.

## BRIEF DESCRIPTION OF DRAWINGS

Figure 1 shows the extraction process of *Cervus nippon* antler.

Figure 2 shows the fractionation of the chloroform extract (CN-C) of *Cervus nippon* antler.

Figure 3 shows a high performance thin layer chromatography (HPTLC) of the fractions obtained in each step of extraction and purification of *Cervus nippon* antler.

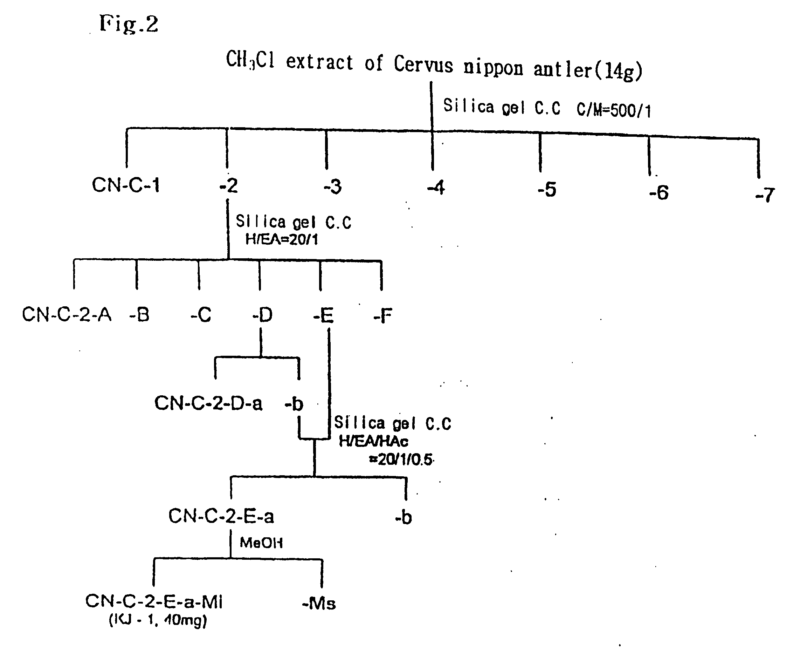

    a: CN-C-2-E-a-Mi of Fig. 2,
    b: CN-C of Fig.2,
    c: CN-C-2 of Fig 2,
    d: CN-C-2-E of Fig. 2,
    e: CN-C-2-E-a of Fig. 2,
    f: CN-C-2-E-a-Mi of Fig. 2

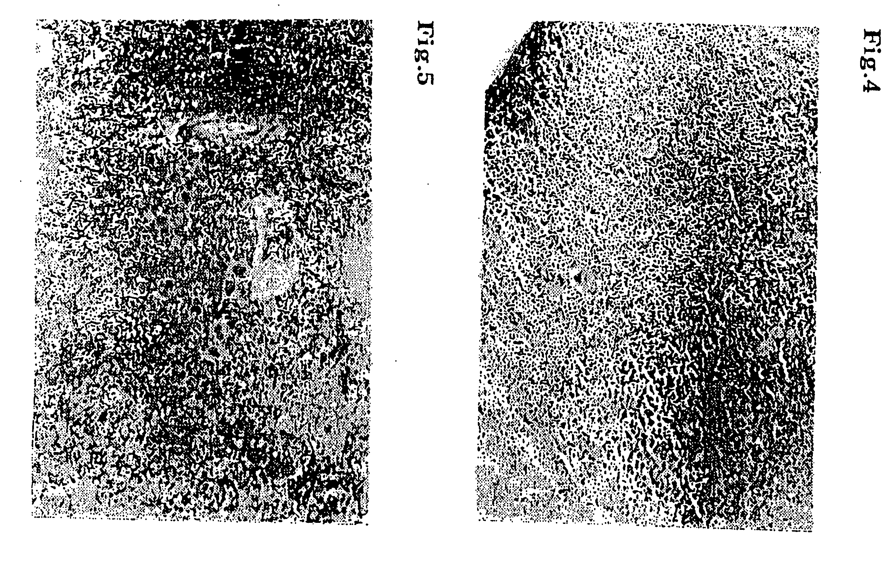

Figure 4 is a photomicrograph of cross section (x 100) of spleen of control mouse.

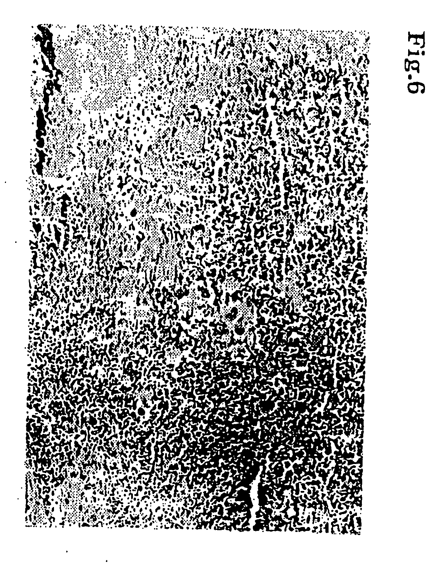

Figure 5 is a photomicrograph of a cross section (x 100) of spleen of the mouse treated with GM-CSF (100U).

Figure 6 is a photomicrograph of a cross secion (×100) of the spleen of the mouse treated with water extract of *Cervus nippon* antler.

## BEST MODE FOR CARRYING OUT THE INVENTION

The present invention is to provide a novel method of extratction from the *Cervus nippon* antler, isolation and purification of the active compounds which have growth-stimulating effects on hematopoietic stem cells and megakaryocytes.

Hematopoiesis is a series of development processs in which bone marrow stern cell (pluripotent stem cell) (HSC) that are capable of both self renewal and production of progenies with potential for differentiation into various blood cell lineages (red cells, granulocytes, and megakaryocytes and lymphocytes). In blood cell system, senescent cells are continually replaced by the progeny of very small number of undifferentiated stem cells which have a capacity for self renewal and differentiation. These stem cells differentiate into and maintain cell population with specific funtional roles. The blood cells produced in the bone marrow are destroyed in a peripheral tissue after certain period of time, and such process is repeated continuously throughout the life of animal and human.

HSCs have two important characteristics; a self-renewal ability to produce the same cells as temselves, and the ability to be differentaited into all systems of lymphatic and hematopoietic cells. Study of hematopoietic cells was initiated from the first discovery that bone marrow suppression can be recovered in quantitative manner by introducing normal bone marrow cells in animals after exposure to a sublethal dose of radiation. Their ability to initiate and sustain long term hematopoiesis is the basis for a successful bone marrow transplant(BMT). As flow cytometry is introduced and monoclonal antibodies on hematopoietic stem cell marker antigen and differentiation antigen are developed, selection of HSCs was possible, and biology and development of hematopoietic cells were better understood.

According to the present invention, biologically active components in the *Cervus nippon* antler can be extracted.

The present invention includes the process of their extraction and fractionation.

The present invention provides a pharmaceutical composition having growth-stimulating activities of hematopoietic stem cells and megakaryocytes containing as an active component adapted to claim 1 chloroform extracts of *Cervus nippon* antler which are obtained by extracting *Cervus nippon* antler with hexane and further extracting the residue of the hexane extract with chloroform, and if necessary, fractionating and purifying the chloroform extract of *Cervus nippon* antler by silica gel column chromatography and high performance thin layer chromatography (HPTLC).

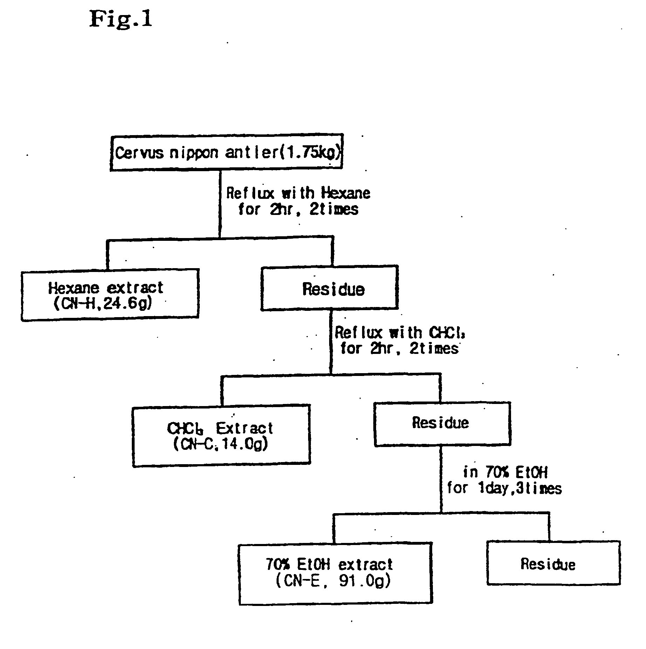

First, the chloroform extracts of *Cervus nippon* antler are obtained first by extracting *Cervus nippon* antler with hexane and further extracting the residue of the hexane extract with chloroform. The process of extraction is shown in Figure 1. The amount of hexane and chloroform used in this extraction process, is the amount enough to impregnate *Cervus nippon* antler. Generally, hexane and chloroform are used in the ratio of 4-5 ℓ with respect to 1kg of *Cervus nippon* antler.

The extract CN-C, a chloroform extract of *Cervus nippon* antler obtained from such extraction process, is fractionated and purified by a series of silica gel column chromatography and high performance thin layer chromatography(HPTLC). The subsequent steps of extract CN-C, chloroform extract of *Cervus nippon* antler, include:

a) step of subjecting the extract CN-C to a silica gel column chromatography using chloroform/methanol as an eluent and subjecting the resulting fraction to HPTLC to give seven extracts, CN-C-1 to CN-C-7, according to the value of $R_f$;
b) step of subjecting the extract CN-C-2 selected from the extracts of step a) to a silica gel column chromatography using hexane/ethyl acetate(10:1, v/v) as an eluent and subjecting the resulting fraction to HPTLC to give six extracts, CN-C-2-A to CN-C-2-F, according to the value of $R_f$;
c) step of subjecting the extract CN-C-2-E selected from the extracts of step b) to a silica gel column chromatography using hexane/ethyl acetate/acetic acid (20:1:0.5. v/v) as an eluent and subjecting the resulting fraction to HPTLC to give two extracts, CN-C-2-E-a and CN-C-2-E-b, according to the value of $R_f$; and
d) step of dissolving the extract CN-C-2-E-a selected from the extracts of step c) in methanol to give methanol-soluble fraction CN-C-2-E-a-Ms and methanol-insoluble fraction CN-C-2-E-a-Mi (development solvent of CN-C-2-E-a-Ms and -Mi : hexane/ethyl acetate/acetic acid = 20:1:0.5. v/v). The fractionation and purification of the chloroform extract CN-C of *Cervus nippon* antler are briefly shown in Figure 2.

The extracts CN-C-2, CN-C-2-E and CN-C-2-E-a obtained from a series of purification processes as set forth above, and the methanol-insoluble fraction CN-C-2-E-a-Mi have growth-stimulating activities of hematopoietic stem cell and megakaryocyte as proved by the subsequent experiments. Therefore, the extract CN-C may be used as a biologically active component in the composition of the present invention.

The growth-stimulating factors of hematopoietic stem cell according to the present invention can be effectively utilized in clinical medicine, for example, diseases requiring improvement of lowed immunity and blood cell function by facilitating the growth of hematopoietic cells, particularly, diseases with bone marrow suppressed by chemotherapy and radiation therapy, e.g., malignant tumors malignant hematological diseases, aplastic anemia, periodic (cyclic) leukoperria immune deficiency disease immune and hematopoietic cells, anemia from end stage renal disease, infection due luekopenia, and poor marrow reserve in elderly population.

The present invention is potential for manufacturing drugs which stimulate hematopoietic stem cells and megakaryocytes. The present invention porvides a pharmaceutical composition containing hematopoietic stem cell and megakaryocyte growth-stimulating substance isolated from the chloroform extracts of *Cervus nippon* antlers or a compound or formula (I) as an active component. When the composition of the present invention is administered for a clinical purpose, it can be formulated into a conventional preparation in pharmaceutical field, for example, preparation for oral administration or injectable preparation. The sterile injectable preparation, for example, water or oil suspension, may be prepared by using a dispersing agent, a wetting agent or a suspending agent in accordance with a known method. The pharmaceutically acceptable solvent which may be used includes water, Ringer's solution and isotropic NaCl solution. The sterile oil may be used as a conventional solvent or suspending medium. Any non-stimulating oil including mono- or di-glyceride may be used for such purpose. Also, the fatty acid such as oleic acid may be used in injectable preparation.

The solid preparation for oral administration may be present in the form of capsule, tablet, pill, powder and granule, particularly capsule and tablet are useful. It is preferable that the tablet and pill are prepared by encapsulation agent. The solid preparation may be prepared by mixing polymer multisugar fraction with one or more inactive diluent such as sucrose, lactose and starch, and lubricant agent such as magnesium stearate, disintegrant, binder and the like.

The dosage of the pharmaceutical composition of the present invention for human being can be suitably determined depending upon kind of extract, fraction or compound to be used as an active component; body weight, diet, sex and healthy condition of subject patient; time and method of administration; amount of excretion, mixing of drug and other condition of subject patient.

The present invention is more specifically explained by the following examples. However, it should be understood that the present invention is not limited to these examples in any manner.

## Example 1

1.75kg of *Cervus nippon* antler (Cervus nippon Temminick var. mantchuricus Swinhoe) is finely breaked and then introduced in beaker. Hexane (about 7 $\ell$) is added in the amount enough to immerse *Cervus nippon* antler, heated for 2 hours at 80°C and then extracted two times and filtered (this filtrate is referred to as extract 1). The extract 1 was distilled under reduced pressure and dried to give extract CN-H (24.6g). The extract 1 was isolated and chloroform(approximately 7 $\ell$) was added to the residue, heated at 70°C for 2 hours and extracted two times and isolated (this solution is referred to as extract 2). The extract 2 was distilled under reduced pressure to give a chloroform extract of *Cervus nippon* antler, extract CN-C (14g). The extract 2 was further isolated and ethanol (about 6 $\ell$) was added to the residue, heated for 1 day at 90°C and extracted three times and filtered. The extract 3 was distilled under reduced pressure to give extract CN-E (91g). The hematopoiesis of the extracts CN-H, CN-C and CN-E thus prepared was evaluated. As a result, the extract CN-C which is considered to have growth-stimulating effects on hematopoietic stem cell was used for the subsequent purification step.

## Example 2

650 ml of a solution mixture of chloroform/methanol(500:1. v/v) was added to 350g of a powder silica gel, allowed to swell and filled with an open column (bed volume = 600 ~ 650 ml). 14g of the extract CN-C obtained in Example 1 was dissolved in a minimum amount (the minium amount for dissolving extracts) of chloroform/methanol (500:1. v/v) and subjected to a silica gel column chromatography. The chloroform/methanol (500:1. v/v) as eluent was added to the column. 50 m$\ell$ of the eluent fraction (void volume: 200m$\ell$) was collected. This was developed with HPTLC (developing solvent: chloroform/ methanol=300:1. v/v), visualized with iodine and isolated according to the value of $R_f$. The values of $R_f$ used were 0.89, 0.63, 0.42, 0.32, 0.18, 0.08 and 0.02, respectively. The seven main fractions thus prepared were isolated. Each of the isolated fractions were distilled under reduced pressure and dried to give the extracts CN-C-1 to CN-C-7. The hematopoiesis of these extracts thus prepared was evaluated as disclosed in the subsequent Experiments 1 to 5. As a result, the extract CN-C-2 which is considered to have growth-stimulating effects on hematopoietic stem cell was used for the subsequent purification step.

## Example 3

The fraction CN-C-2 (380mg) having excellent hematopoiesis among the fractions purified in Example 2 was taken. 50 ml of a solution mixture of hexane/ethyl acetate(50:1. v/v) was added to 20g of a powder silica gel, allowed to swell and filled with an open column (bed volume = 100ml). 380g of the extract CN-C was dissolved in a minimum amount of chloroform/methanol (50:1. v/v) and subjected to a silica gel column chromatography. The chloroform/memanol (500:1. v/v) as eluent was added thereto in the same manner as in Example 2 and then 15m$\ell$ of the eluent fraction was collected. This fraction was developed with HPTLC (developing solvent: chloroform/methanol=50:1. v/v), visualized with iodine and isolated into six parts according to the value of $R_f$. The values of $R_f$ used were 0.76, 0.62, 0.43, 0.38, 0.32 and 0.11, respectively. The fractions thus prepared were isolated. Each of the isolated fractions were distilled under reduced pressure and dried to give the extracts CN-C-2-A to CN-C-2-F. The hematopoiesis of these six extracts was evaluated as disclosed in Experiments 1 to 5. As a result, the extract CN-C-2-E which is considered to have growth-stimulating effects on hematopoietic stem cell was used for the subsequent purification step.

## Example 4

The fraction CN-C-2-E (70mg) having excellent hematopoiesis among the fractions purified in Example 3 was taken. 10 ml of a solution mixture of hexane/ethyl acetate/acetic acid(20:1:0.5. v/v) was added to 3.5g of a powder silica gel, allowed to swell and filled with column (bed volume = 24ml). 70g of the extract CN-C-2-E was dissolved in a minimum amount of hexane/ethyl acetate/acetic acid(20:1:0.5. v/v) and then subjected to a silica gel column. The eluent was added thereto in the same manner as in Example 3 and then 10 ml of eluent fraction was collected. This fraction was developed with HPTLC (developing solvent: hexane/ethyl acetate/acetic acid=20:1:0.5. v/v), visualized with iodine and then classified into two parts according to the value of $R_f$. The values of $R_f$ used were 0.34 and 0.16, respectively. These two extracts were expressed as CN-C-2-E-a and CN-C-2-E-b. The hematopoiesis of the extracts thus prepared was evaluated as disclosed in Experiments 1 to 5. As a result, the extract CN-C-2-E-a which is considered to have growth-stimulating effects on hematopoietic stem cell was used in the subsequent purification step.

## Example 5

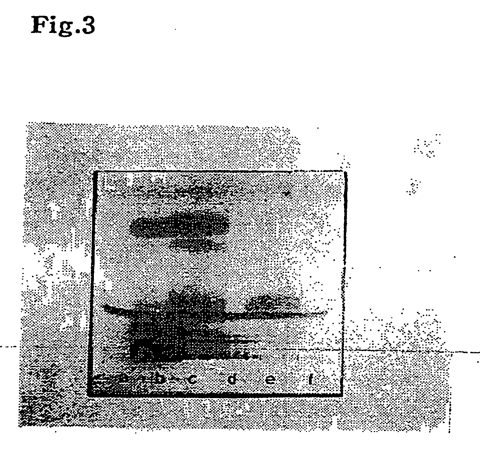

The first part, extract CN-C-2-E-a (62mg), among the parts isolated in Example 4 was taken. To this extract CN-C-2-E-a was added 5ml of methanol which was then isolated into methanol-soulble part (extract CN-C-2-E-a-Ms; $R_f$ = 0.22)) and methanol-insoluble part (extract CN-C-2-E-a-Mi; $R_f$ = 0.24) to obtain 20mg of extract CN-C-2-E-a-Ms and 40mg of extract CN-C-2-E-a-Mi. The HPTLC results on respective active components obtained in Examples 1 to 5 is shown in Figure 3. It can be seen from Figure 3 that the band in extract CN-C is the same in the course of purification. Therefore, it can be seen that the active parts is not denatured or disappared in the course of purification. At first, the band hiden due to the other part was deeply develpoed as the purification was proceeded.

**Experiment 1**

**Colony-Forming Unit in Culture(CFUc) assay:**

The ability of bone marrow stem cells to form colonies *in vitro* culture was evaluated in a single layer methyl cellulose culture system.

1-1. Separation of bone marrow cell of mouse

Six to 8 weeks old C57B 1/6 male mice bred in specific pathogene free (SPF) condition were used. The bone marrow of mouse was separated by washing with RPMI 1640 nutrient medium (Gibco #430-1800) from mouse femora and tibiae. The separated bone marrow was filtered to obtain mononuclear cells, washed three times with RPMI 1640 medium, and then the number of cells were counted. Stem cell-rich fraction was prepared by discontinuous percol gradient centrifugation. The cell at the interface between the density of 1.063 and 1.075 solution were collected.

1-2. Preparation of Sample

All extract samples of *Cervus nippon* antler were dissolved in dimethylsulfoxide (DMSO) to the concentration of 100mg/ml. This solution was used as a stock solution and used for evalution of hematopoiesis.

1-3. Analysis of CFU-C (colony-forming unit in culture)

The cells, collected in the method of the item 1-1 were plated with $1.2 \times 10^4$ cell/well in 1.1% methylceiluose plate which was cultured in $CO_2$-incubator at 37°C. After one week of culture, the number of colony was counted. The *Cervus nippon* antler extracts CN-C, CN-C-2, CN-C-2-E, CN-C-2-E-a and CN-C-2-E-a-Mi separatedly added to the stem cells and compared with control treated with 1% PBS only. The result of colony forming efficiency is shown in Table 2. The colony forming efficiency (CFE) was obtained according to the following equation.

$$CFE = (total\ number\ of\ colony\ in\ sample)\ /\ (total\ number\ of\ colony\ in\ control\ group)$$

Table 2.

| Sample | colony forming efficiency (0.001mg/ml) | number of colony in sample/number of colony in control group |
|---|---|---|
| Extract CN-C | 1.11 | 171/154 |
| Extract CN-C-2 | 1.22 | 188/154 |
| Extract CN-C-2-E | 1.23 | 189/154 |
| Extract CN-C-2-E-a | 1.29 | 199/154 |
| Extract CN-C-2-E-a-Mi | 1.30 | 200/154 |

As can be seen from the result in Table 2, the chloroform extract of *Cervus nippon* antler or respective fraction purified therefrom according to the present invention exhibit excellent colony forming efficiency and thus it may be concluded that they have stimulating effects on hematopoietic stem cells.

**Claims**

1. A pharmaceutical composition having growth-stimulating activities of hematopoietic stem sells and megakaryocytes containing as an active component chloroform extracts of *Cervus nippon* antler (hereinafter referred to as "extract CN-C") which are obtained by extracting *Cervus nippon* antler with hexane and further extracting the residue of the hexane extract with chloroform, and if necessary, fractionating and purifying the chloroform extract of *Cervus nippon* antler by silica gel column chromatography and high performance thin layer chromatography (HPTLC).

2. The composition of claim 1, comprising as an active component a fraction CN-C-2 obtained by subjecting the extract CN-C to columnt chromatography and HPTLC using chloroform/methanol (500/l. v/v) as an eluent.

3. The composition of claim 2, comprising as an active component a fraction CN-C-2-E obtained by subjecting the extract CN-C-2 to column chromatography an HPTLC using hexanlacetaldehyde (20/l. v/v) as an eluent.

4. The composition of claim 3, containing as an active component a fraction CN-C-2-E-a obtained by subjecting the extract CN-C-2-E to column chromatography and HPTLC using hexan/acetic acid (20/l/0.5 v/v) as an eluent.

5. The composition of claim 4, comprising as an active component a fraction CN-C-2-E-a-Mi obtained by dissolving the extract CN-C-2-E-a in methanol.

6. A process for preparing a chloroform extract of *Cervus nippon* antler as defined in any one of claims 1 to 5 which comprises extracting *Cervus nippon* antler with hexane and further extracting the residue of the hexane extract with chloroform to give the chloroform extract of *Cervus nippon* antler, and if necessary, fractionating and purifying the chloroform extract by silica gel column chromatrography and HPTLC.

7. The composition according to anyone of the claims 1 to 5 wherein it is administrable orally or parenterally.

**Patentansprüche**

1. Arzneimittel mit wachstumsstimulierenden Aktivitäten von hämotopoietischen Stammzellen und Megakaryozyten, die als Wirkbestandteil Chloroformextrakte vom Geweih des *Cervus nippon* (hier nachstehend als "Extrakt CN-C" bezeichnet) enthalten, die durch Extrahieren des Geweihs von *Cervus nippon* mit Hexan und weiteres Extrahieren des Rückstands des Hexanextrakts mit Chloroform und gegebenenfalls Fraktionieren und Reinigen des Chloroformextrakts des Geweihs von *Cervus nippon* durch Silicagelsäulenchromatografie und Hochleistungsdünnschichtchromatographie (HPTLC) erhalten werden.

2. Arzneimittel nach Anspruch 1, umfassend als Wirkbestandteil eine Fraktion CN-C-2, die durch Unterziehen des Extrakts CN-C einer Säulenchromatographie und HPTLC unter Verwendung von Chloroform/Methanol (500/1 V/V) als Eluent erhalten wird.

3. Arzneimittel nach Anspruch 2, umfassend als Wirkbestandteil eine Fraktion CN-C-2-E, die durch Unterziehen des Extrakts CN-C-2 einer Säulenchromatographie und HPTLC unter Verwendung von Hexan/Acetaldehyd (20/l V/V) als Eluent erhalten wird.

4. Arzneimittel nach Anspruch 3, umfassend als Wirkbestandteil eine Fraktion CN-C-2-E-a, die durch Unterziehen des Extrakts CN-C-2-E einer Säulenchromatographie und HPTLC unter Verwendung von Hexan/Essigsäure (20/0,5 V/V) als Eluent erhalten wird.

**Revendications**

1. Upe composition pharmaceutique présentant des activités de stimulation de la croissance des cellules souches hématopoïétiques et des mégacaryocytes, contenant comme principe actif des extraits au chloroforme de merrain de *Cervus Nippon* (ci-après désigné "extrait CN-C") qui sont obtenus par extraction à l'hexane de merrain de *Cervus*

*Nippon* et ensuite extraction au chloroforme du résidu de l'extrait à l'hexane et, si nécessaire, fractionnement et purification de l'extrait au chloroforme de merrain de *Cervus Nippon* par chromatographie sur colonne de gel de silice et chromatographie sur couche mince à hautes performances (HPTLC).

2. La composition de la revendication 1, comprenant comme principe actif une fraction CN-C-2 obtenue en soumettant l'extrait CN-C à une chromatographie sur colonne et à une HPTLC en utilisant comme éluant du chloroforme/méthanol (500/1 v/v).

3. La composition de la revendication 2, comprenant comme principe actif une fraction CN-C-2-E obtenue en soumettant l'extrait CN-C-2 à une chromatographie sur colonne et à une HPTLC en utilisant comme éluant de l'hexane/acétaldéhyde (20/1 v/v).

4. La composition de la revendication 3, comprenant comme principe actif une fraction CN-C-2-E-a obtenue en soumettant l'extrait CN-C-2-E à une chromatographie sur colonne et à une HPTLC en utilisant comme éluant de l'hexane/acide acétique (20/1/0,5 v/v).

**Fig.1**

```
              Cervus nippon antler(1.75kg)
                          |
              Reflux with Hexane
                 for 2hr, 2times
            _____|_____
           |                       |
   Hexane extract              Residue
   (CN-H,24.6g)                   |
                         Reflux with CHCl₃
                            for 2hr, 2times
                        _____|_____
                       |               |
                CHCl₃ Extract       Residue
                (CN-C,14.0g)           |
                               in 70% EtOH
                              for 1day,3times
                            _____|_____
                           |               |
                   70% EtOH extract     Residue
                    (CN-E, 91.0g)
```

# Fig.2

CH₃Cl extract of Cervus nippon antler(14g)

$CH_3Cl$ extract of Cervus nippon antler(14g)

| Silica gel C.C   C/M=500/1

CN-C-1    -2    -3    -4    -5    -6    -7

Silica gel C.C
H/EA=20/1

CN-C-2-A   -B   -C   -D   -E   -F

CN-C-2-D-a   -b

Silica gel C.C
H/EA/HAc
≈20/1/0.5

CN-C-2-E-a                    -b

MeOH

CN-C-2-E-a-Ml            -Ms
(KJ - 1, 10mg)

EP 1 061 932 B1

Fig.3

Fig.4

Fig.5

Fig.6